# EUROPEAN PATENT APPLICATION

(11) **EP 2 695 880 A1**
(43) Date of publication of application: **12.02.2014**
(21) Application number: 13739925.9
(22) Date of filing: 06.05.2013
(51) Int. Cl.: C07C 253/32, C07C 255/09, B01J 31/12, H01M 10/0567

(54) **NONAQUEOUS ELECTROLYTE SOLUTION FOR LITHIUM SECONDARY BATTERY, AND LITHIUM SECONDARY BATTERY CONTAINING SAME**

(30) Priority: 22.05.2012 KR 20120054304
(71) Applicant: LG Chem, Ltd., Seoul 150-721 (KR)
(72) Inventor: LEE, Byoung Bae, Yuseong-gu, Daejeon 305-721 (KR); OH, Jae Seung, Songpa-gu, Seoul 138-911 (KR); HONG, Yeon Suk, Yuseong-gu, Daejeon 305-340 (KR); KIM, Min Ah, Yuseong-gu, Daejeon 305-509 (KR)
(74) Representative: Goddar, Heinz J.
(86) International application number: PCT/KR2013/003907
(87) International publication number: WO 2013/176421

(57) **Abstract**

A method of preparing a tricyanoalkoxy alkane compound is disclosed. The tricyanoalkoxy alkane compound is prepared by reacting an alcohol compound including at least three hydroxyl groups and a nitrile compound in the presence of a potassium alkoxide catalyst. A non-aqueous electrolyte solution comprising the tricyanoalkaxy alkane compound and a lithium secondary battery also are disclosed. The swelling of the battery may be prevented.

## Description

### [Technical Field]

The present invention relates to a non-aqueous electrolyte solution for a lithium secondary battery comprising a tricyanoalkoxy alkane compound prepared by reacting a nitrile compound and an alcohol compound having at least three hydroxyl groups in the presence of a potassium alkoxide catalyst, and a lithium secondary battery comprising the same, in which the swelling of the battery may be prevented.

### [Background Art]

Recently, technology on an energy storage system attracts much concern as the application field of an energy storage technique is enlarged to a cellular phone, a camcorder, and a notebook PC and even to an electric vehicle. Particularly, demands on a high energy density battery used as a power source in an electronic device in the energy storage technique are increasing day by day. A lithium secondary battery is considered as a battery satisfying the demands very well, and researches on the lithium secondary battery is actively conducted.

Among the presently used secondary batteries, a lithium secondary battery developed on the early 1990s includes a negative electrode of a carbon material for absorbing and emitting lithium ions, a positive electrode including a lithium-containing oxide, etc. and a non-aqueous electrolyte including an appropriate amount of a lithium salt dissolved in an organic solvent.

The organic solvent for the non-aqueous electrolyte comprises ethylene carbonate, propylene carbonate, dimethoxyethane, gamma butyrolactone, N,N-dimethyl formamide, tetrahydrofuran, acetonitrile, etc.

However, when the battery is stored at a high temperature for a long time, the non-aqueous electrolyte using the organic solvent may be oxidized to generate a gas and to deform the structure of the battery. Otherwise, the internal heating of a battery by an overcharge or an over discharge may become greater, and an internal short may be generated to induce an ignition or an explosion of the battery.

In order to solve the above-described defects, various attempts for improving battery stability at a high temperature have been tried including (1) employing a porous polyolefin-based separator having a high melting point so as not to melt at a high temperature or (2) mixing an insoluble solvent or a nitrile compound as an additives in an electrolyte.

The nitrile compound may be commonly prepared by a cyanoethylation reaction, in which a step of reacting a compound including a hydroxyl-group with acrylonitrile in a water medium is performed. In the cyanoethylation reaction, a catalyst such as a caustic alkali, a quaternary ammonium salt, and sodium hydroxide etc. may be used. Among the catalysts, sodium hydroxide is economic and liable for a synthesis, and so is most widely used. However, in the cyanoethylation reaction, water reacts with the acrylonitrile prior to the compound having the hydroxyl group to form a cyanoethylated compound. Then, this cyanoethylated compound reacts with the acrylonitrile again to produce a by-product of bis(2-cyanoethyl)ether.

In order to prevent the production of the by-product, the amount of the acrylonitrile consumed in the reaction may be controlled or the concentration of the acrylonitrile may be controlled in the reactants by slowly dropping the acrylonitrile. In this case, however, the remaining amount of the hydroxyl compound among the reactants may be increased to deteriorate the quality. In addition, when the reaction is conducted in a non-aqueous condition to prevent the cyanoethylation due to water, polymerization of the acrylonitrile may proceed in the presence of the naustic alkali or an organic base. Due to the polymerization, the color of the reactant may change.

Meanwhile, Patent Literature 1 discloses a method of preparing a cyanoethyl compound by using lithium hydroxide as a reaction catalyst in a non-aqueous condition to overcome the above-described defects. According to this method, the production of bis(2-cyanoethyl)ether may be prevented and the coloring due to the polymerization of the acrylonitrile may be decreased. However, the solubility of the lithium hydroxide may be decreased, and a reaction time may be increased.

Patent Literature 2 discloses a method of synthesizing 1,3,6-tricyanohexane compound by reacting adiponitrile and t-butyl alcohol in the presence of a base catalyst, for example, potassium or sodium alcoholate of an aliphatic alcohol.

Non-patent Literature 1 discloses a method of preparing a tricyano compound by using sodium methoxide.

However, according to the above-described methods, the yield is low and a large amount of by-products is produced.

### [Prior Art]

### [Patent Literature]

(Patent Literature 1) Japanese Patent Application Laid-open Publication No. 3946825
(Patent Literature 2) Japanese Patent Publication No. 53-135926

### [Non-patent Literature]

(Non-patent Literature 1) H. A. Bruson and T.W.Riener, J. Am. Chem. Soc. 65, 23(1943), "Synthesis and characterization of new diisocyanide rhenium complexes. Comparison with the homoleptic 99mTC complex"

### [Disclosure]

### [Technical Problem]

An object of the present invention is to provide a method of preparing a tricyanoalkoxy alkane compound with a decreased preparation time and increased yield.

Another object of the present invention is to provide a non-aqueous electrolyte solution comprising the tricyanoalkoxy compound prepared by the above method as an additive in order to largely prevent a battery from the swelling due to a gas generated during storing at a high temperature.

A further object of the present invention is to provide a secondary battery including the non-aqueous electrolyte.

### [Technical Solution]

According to the present invention, a method of preparing a tricyanoalkoxy alkane compound is provided. The compound is prepared by reacting an alcohol compound including at least three hydroxyl groups represented by Chemical Formula 2 and a nitrile compound represented by Chemical Formula 3 in the presence of a potassium alkoxide catalyst represented by Chemical Formula 1.

[Chemical Formula 1] RO-K

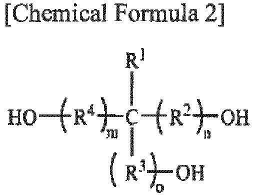

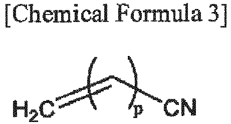

In which, R represents a linear or branched C₁-C₅ alkyl group, R¹ represents hydrogen or a linear or branched C₁-C₃ alkyl group, R², R³ and R⁴ individually represent a linear chain or branched C₁-C₅ alkylene group, each of m, n and o represents an integer of 0 to 5, and p represents an integer of 1 to 10.

### [Advantageous Effects]

According to the method of preparing a tricyanoalkoxy alkane compound in the present invention, the preparation time of the tricyanoalkoxy alkane compound may be decreased, and a yield may be increased by decreasing the production of by-products. In addition, through comprising the tricyanoalkoxy alkane compound prepared by the present invention in an electrolyte, a swelling of a battery due to a gas generated during storing at a high temperature may be largely prevented. Thus, a lithium battery having a good charge/discharge performance may be provided.

### [Best Mode]

Hereinafter, the present invention will be described in detail. All terms or words used herein should not be defined and interpreted as having common or dictionary meanings, but should be interpreted as being consistent with their meanings and concepts of the technical spirit of the present invention based on the principle that the inventors could appropriately define the concept of the terms to explain the invention by his best way.

According to the present invention, a method of preparing a tricyanoalkoxy alkane compound is provided. The compound is prepared by reacting an alcohol compound including at least three hydroxyl groups represented by Chemical Formula 2 and a nitrile compound represented by Chemical Formula 3 in the presence of a potassium alkoxide catalyst represented by Chemical Formula 1.

[Chemical Formula 1] RO-K

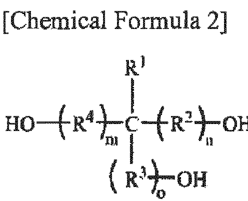

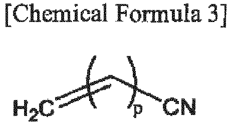

In which, R represents a linear or branched C₁-C₅ alkyl group, R¹ represents hydrogen or a linear or branched C₁-C₃ alkyl group, R², R³ and R⁴ individually represent a linear chain or branched C₁-C₅ alkylene group, each of m, n and o represents an integer of 0 to 5, and p represents an integer of 1 to 10.

In example embodiments, the potassium alkoxide catalyst represented by Chemical Formula 1 may be at least one catalyst selected from the group consisting of, for example, potassium methoxide, potassium ethoxide, potassium t-butoxide and potassium t-pentoxide. The amount of the potassium alkoxide catalyst may be 0.01 to 5 parts by weight based on 100 parts by weight of the alcohol compound including at least three hydroxyl groups. When the amount of the potassium alkoxide catalyst is less than 0.01 parts by weight, the reaction may not proceed completely. When the amount of the potassium alkoxide exceeds 5 parts by weight, a large amount of by-products may be produced.

In addition, the alcohol compound including at least three hydroxyl groups represented by Chemical Formula 2 may be at least one compound selected from the group consisting of 1,2,3-propanetriol, 1,2,4-butanetriol, 1,1,1-methylene ethanetriol, 1,1,1-methylene propanetriol, 3-methyl-1,3,5-pentanetriol, 1,2,7-heptanetriol, 1,2,6-hexanetriol and 1,2,5-pentanetriol.

In example embodiments, the nitrile compound represented by Chemical Formula 3 may be at least one selected from the group consisting of acrylonitrile, allyl cyanide, crotononitrile, 3-methyl-3-butanenitrile, 2-pentenenitrile and 3-pentenenitrile. The nitrile compound represented by Chemical Formula 3 may be included by an amount of 150 to 400 parts by weight, for example, 150 to 300 parts by weight, or 300 parts by weight, based on 100 parts by weight of the alcohol compound including at least three hydroxyl groups. When the amount of the nitrile compound is less than 150 parts by weight, unreacted products may be obtained. When the amount of the nitrile compound exceeds 400 parts by weight, the nitrile compound may remain.

The method of preparing the tricyanoalkoxy alkane compound according to the present invention, may comprise a step of stirring a potassium alkoxide catalyst represented by Chemical Formula 1 and an alcohol compound including at least three hydroxyl groups represented by Chemical Formula 2 in a non-aqueous solvent or a solvent free condition, at a temperature from room temperature to 40°C; and slowly dropping the nitrile compound represented by Chemical Formula 3.

In addition, the tricyanoalkoxy alkane compound prepared by the method described above may be represented by following Chemical Formula 4.

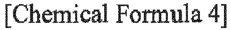

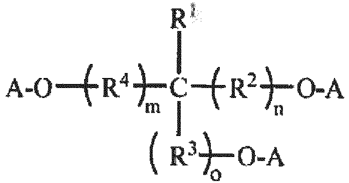

In which, R¹ represents hydrogen or a linear or branched C₁-C₃ alkyl group, R², R³ and R⁴ individually represent a linear or branched C₁-C₅ alkylene group, A represents an -R⁵-CN group, R⁵ represents a linear or branched C₂-C₁₁ alkylene, and each of m, n and o represents an integer of 0 to 5.

Particularly, the tricyanoalkoxy alkane compound prepared by the method of the present invention and represented by Chemical Formula 4 may be one or more compound selected from the group consisting of 1,2,3-tris(2-cyanoethoxy)propane, 1,2,4-tris(2-cyanoethoxy)butane, 1,1,1-tris(cyanoethoxy methylene)ethane, 1,1,1-tris(cyanoethoxy methylene)propane, 3-methyl 1,3,5-tris(cyanoethoxy)pentane, 1,2,7-tris(cyanoethoxy)heptane, 1,2,6-tris(cyanoethoxy)hexane and 1,2,5-tris(cyanoethyoxy)pentane.

By using the potassium alkoxide catalyst in the method of preparing the tricyanoalkoxy alkane compound according to the present invention, bis(2-cyanoethyl)ether may not be produced, and the polymerization of the acrylonitrile and the coloring may not be generated. Thus, the generation of impurities may be decreased.

In addition, an electrolyte solution for a lithium secondary battery including the tricyanoalkoxy alkane compound prepared by the method of the present invention, a lithium salt and a non-aqueous solvent is provided.

In example embodiments, the lithium salt may include Li⁺ cation and at least one anion selected from the group consisting of F⁻, Cl⁻, Br⁻, I⁻, NO₃⁻, N(CN)₂⁻, BF₄⁻, ClO₄⁻, PF₆⁻, (CF₃)₂PF₄⁻, (CF₃)₃PF₃⁻, (CF₃)₄PF₂⁻, (CF₃)₅PF⁻, (CF₃)₆P⁻, CF₃SO₃⁻, CF₃CF₂SO₃⁻, SCN⁻, (CF₃CF₂SO₂)₂N⁻, (CF₃SO₂)₂N⁻, (FSO₂)₂N⁻, CF₃CF₂(CF₃)₂CO⁻, (CF₃SO₂)₂CH⁻, (SF₆)₃C⁻, (CF₃SO₂)₃C⁻, CF₃(CF₂)₇SO₃⁻, CF₃CO₂⁻ and CH₃CO₂⁻.

In addition, the non-aqueous solvent may be a mixture solution of a cyclic carbonate and a linear carbonate. Particularly, at least one selected from the group consisting of propylene carbonate, ethylene carbonate, diethy carbonate, dimethyl carbonate, ethyl methyl carbonate, methyl propyl carbonate, dipropyl carbonate, dimethyl sulfoxide, acetonitrile, dimethoxyethane, diethoxyethane, vinylene carbonate, sulfolane, gamma-butyrolactone, propylene sulfite and tetrahydrofuran may be used. Particularly, the propylene carbonate and the ethylene carbonate of the cyclic carbonate among the carbonate-based organic solvents are organic solvents having a high viscosity and having a high dielectricity. Thus, the dissociation of the lithium salt in the electrolyte may be good. When an appropriate amount of the cyclic carbonate is mixed with the linear carbonate having a low viscosity and dielectricity, an electrolyte having a high electric conductivity may be obtained.

The electrolyte solution according to the present invention may be usefully applied for manufacturing an electrochemical device such as a lithium secondary battery.

Particularly the lithium secondary battery is provided by injecting the non-aqueous electrolyte solution of the present invention into a case of lithium secondary battery including the electrode assembly. The electrode assembly includes a positive electrode, a negative electrode, and a separator disposed between the positive electrode and the negative electrode. In this case, commonly used positive electrode, negative electrode and separator may be used for constituting the electrode assembly.

As an active material for the positive electrode, a transition metal oxide containing lithium may be preferably used. For example, at least one or a mixture of two or more of LiCoO₂, LiNiO₂, LiMnO₂, LiMn₂O_{4,}, Li(NiₐCo_{b}Mn_{c})O₂, (0<a<1, 0<b<1, 0<c<1, a+b+c=1), LiMn₂O₄LiNi_{1-y}Co_{y}O₂, LiCo_{1-y}Mn_{y}O₂, LiNi_{1-y}Mn_{y}O₂ (0<y<1), Li(NiₐMn_{b}Co_{c})O₄ (0<a<2, 0<b<2, 0<c<2, a+b+c=2), LiMn_{2-z}Ni_{z}O₄, LiMn_{2-z}Co_{z}O₄ (0<z<2), LiCoPO₄ and LiFePO₄ may be used. Beside the above described oxides, a sulfide, a selenide and a halide, etc. may be used.

As an active material for the negative electrode, a carbon material, a lithium metal, silicon, tin, etc. may be commonly used for absorbing and emitting lithium ions. In addition, a metal oxide such as TiO₂ and SnO₂, having a potential of 2V or less with respect to lithium may also be used. The carbon material may be preferably used, and both of a low crystalline carbon and a highly crystalline carbon may be used as the carbon material. As the low crystalline carbon material, soft carbon and hard carbon may be typically included. As the highly crystalline carbon material, natural graphite, kish graphite, pyrolytic carbon, mesophase pitch based carbon fiber, meso-carbon microbeads, mesophase pitches and a high temperature clacined carbon such as petroleum or coal tar pitch derived cokes may be typically included. In this case, a binding agent may be included in the negative electrode, and the binding agent may include various kinds of binder polymers such as vinylidene fluoride-hexafluoropropylene copolymer (PVDF-co-HFP), polyvinylidene fluoride, polyacrylonitrile, polymethyl methacrylate, etc.

In addition, the separator may be used commonly used porous polymer film. The porous polymer film comprises alone or laminate porous polymer film manufactured by using an olefinic polymer such as an ethylene homopolymer, a propylene homopolymer, an ethylene/butane copolymer, an ethylene/butene copolymer, an ethylene/hexene copolymer, an ethylene/hexane copolymer and an ethylene/methacrylate copolymer. In addition, a commonly used porous non woven fabric, for example, a high melting point glass fiber, and a non woven fabric of a polyethylene terephthalate fiber, etc. may be used as the separator, without specific limitation.

The shape of the lithium secondary battery according to the present invention includes a polygonal shape or a cylindrical shape using a can, a pouch shape or a coated shape, etc. without specific limitation.

### [Mode for Intention]

Hereinafter, reference will now be made in detail to the preferred embodiments of the present invention. However, various modifications on the preferred embodiments of the present invention will be made, and the scope of the present invention will not be limited to the following embodiments.

### [Examples]

Hereinafter, reference will now be made in detail to the preferred embodiments of the present invention. However, various modifications on the preferred embodiments of the present invention will be made, and the scope of the present invention will not be limited to the following embodiments.

### Example 1

92.09g of 1,2,3-propanetriol as a compound having a hydroxyl group and 0.2g of potassium t-butoxide catalyst were added into a flask, and then heated to 40°C and stirred. 159.18g of acrylonitrile was dropwisely added for 1 hour and stirred for 5 hours. The reaction product was extracted using 200g of methylene chloride and 500g of distilled water. The extraction process was conducted once more, and a methylene chloride solution was separated and distilled under a reduced pressure to produce a 1,2,3-tris(2-cyanoethoxy)propane compound as a colorless transparent compound (purity measured by a gas chromatography was 99%).

### Comparative Example 1

9.2g of 1,2,3-propanetriol as a compound having a hydroxyl group and 160 ml of a 2% aqueous sodium hydroxide solution were added into a flask, and then heated to 40°C and stirred. 15.9g of acrylonitrile was dropwisely added for 1 hour and stirred for 3 hours. The reaction product was extracted using 200g of methylene chloride and 500g of distilled water. The extraction process was conducted once more, and a methylene chloride solution was separated and distilled under a reduced pressure to produce a 1,2,3-tris(2-cyanoethoxy)propane compound (purity measured by a gas chromatography was 71 % for the 1,2,3-tris(2-cyanoethoxy)propane compound and 29% for a by-product, bis(2-cyanoethyl)ether).

### Comparative Example 2

9.2g of 1,2,3-propanetriol as a compound having a hydroxyl group and 0.1g of sodium hydroxide were added into a flask, and then heated to 40°C and stirred. 15.9g of acrylonitrile was dropwisely added for 2 hours. After 1 hour from the start of the dropping, the color of the reactant slowly was changed, and after completing the dropping, the reactant was changed into black with a precipitation. Desired cyanoethyl compound was not confirmed through measuring by a gas chromatography.

### Comparative Example 3

The same procedure described in Example 1 was conducted except for using 5.6g (6 parts by weight) of a potassium t-butoxide catalyst to produce a 1,2,3-tris(2-cyanoethoxy)propane compound (purity measured by a gas chromatography was 71 % for the 1,2,3-tris(2-cyanoethoxy)propane compound and 29% for a by-product, bis(2-cyanoethyl)ether).

## Claims

1. A method of preparing a tricyanoalkoxy alkane compound, the method comprising reacting an alcohol compound including at least three hydroxyl groups represented by Chemical Formula 2 and a nitrile compound represented by Chemical Formula 3 in the presence of a potassium alkoxide catalyst represented by Chemical Formula 1,
[Chemical Formula 1] RO-K 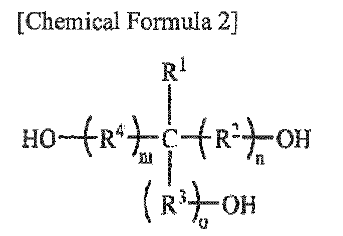 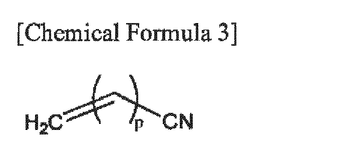
in which, R represents a linear or branched C₁-C₅ alkyl group, R¹ represents hydrogen or a linear or branched C₁-C₃ alkyl group, R², R³ and R⁴ individually represent a linear chain or branched C₁-C₅ alkylene group, each of m, n and o represents an integer of 0 to 5, and p represents an integer of 1 to 10.

2. The method according to claim 1, wherein the potassium alkoxide catalyst represented by Chemical Formula 1 is at least one catalyst selected from the group consisting of potassium methoxide, potassium ethoxide, potassium tert-butoxide and potassium tert-pentoxide.

3. The method according to claims 1 or 2, wherein an amount of the potassium alkoxide catalyst is 0.01 to 5 parts by weight based on 100 parts by weight of the alcohol compound including at least three hydroxyl groups.

4. The method according to claim 1, wherein the alcohol compound including at least three hydroxyl groups represented by Chemical Formula 2 is at least one compound selected from the group consisting of 1,2,3-propanetriol, 1,2,4-butanetriol, 1,1,1-methylene ethanetriol, 1,1,1-methylene propanetriol, 3-methyl-1,3,5-pentanetriol, 1,2,7-heptanetriol, 1,2,6-hexanetriol and 1,2,5-pentanetriol.

5. The method according to claim 1, wherein the nitrile compound represented by Chemical Formula 3 is at least one selected from the group consisting of acrylonitrile, allyl cyanide, crotononitrile, 3-methyl-3-butanenitrile, 2-pentenenitrile and 3-pentenenitrile.

6. The method according to claim 1, wherein the nitrile compound represented by Chemical Formula 3 is used by an amount of 150 to 400 parts by weight based on 100 parts by weight of the alcohol compound including at least three hydroxyl groups.

7. The method according to claim 1, wherein the method comprises
stirring the potassium alkoxide catalyst represented by Chemical Formula 1 and the alcohol compound including at least three hydroxyl groups represented by Chemical Formula 2 in a non-aqueous solvent or a solvent free condition, at a temperature from room temperature to 40°C; and
slowly dropping the nitrile compound represented by Chemical Formula 3 into the mixture solution of the potassium alkoxide catalyst and the alcohol compound including at least three hydroxyl groups.

8. A tricyanoalkoxy alkane compound prepared by the method described in claim 1 and represented by following Chemical Formula 4, 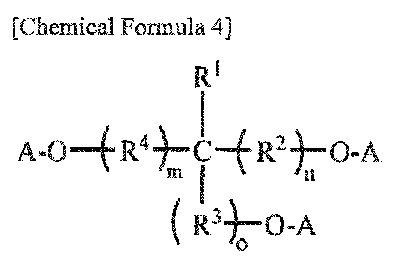 in which, R¹ represents hydrogen or a linear or branched C₁-C₃ alkyl group, R², R³ and R⁴ individually represent a linear or branched C₁-C₅ alkylene group, A represents an -R⁵-CN group, R⁵ represents a linear or branched C₂-C₁₁ alkylene, and each of m, n and o represents an integer of 0 to 5.

9. The compound according to claim 8, wherein the tricyanoalkoxy alkane compound is one or more selected from the group consisting of 1,2,3-tris(2-cyanoethoxy)propane, 1,2,4-tris(2-cyanoethoxy)butane, 1,1,1-tris(cyanoethoxy methylene)ethane, 1,1,1-tris(cyanoethoxy methylene)propane, 3-methyl 1,3,5-tris(cyanoethoxy)pentane, 1,2,7-tris(cyanoethoxy)heptane, 1,2,6-tris(cyanoethoxy)hexane and 1,2,5-tris(cyanoethyoxy)pentane.

10. An electrolyte solution for a lithium secondary battery comprising a lithium salt and a non-aqueous solvent, the electrolyte solution further comprising the tricyanoalkoxy alkane compound described in claim 8.

11. The electrolyte solution according to claim 10, wherein the lithium salt comprises Li⁺ cation and at least one anion selected from the group consisting of F⁻, Cl⁻, Br⁻, I⁻, NO₃⁻, N(CN)₂⁻, BF₄⁻, ClO₄⁻, PF₆⁻, (CF₃)₂PF₄⁻, (CF₃)₃PF₃⁻, (CF₃)₄PF₂⁻, (CF₃)₅PF⁻, (CF₃)₆P⁻, CF₃SO₃⁻, CF₃CF₂SO₃⁻, SCN, (CF₃CF₂SO₂)₂N⁻, (CF₃SO₂)₂N⁻, (FSO₂)₂N⁻, CF₃CF₂(CF₃)₂CO⁻, (CF₃SO₂)₂CH⁻, (SF₆)₃C⁻, (CF₃SO₂)₃C⁻, CF₃(CF₂)₇SO₃⁻, CF₃CO₂⁻ and CH₃CO₂⁻.

12. The electrolyte solution according to claim 10, wherein the non-aqueous solvent is a mixture solution of a cyclic carbonate and a linear carbonate.

13. The electrolyte solution according to claim 12, wherein the non-aqueous solvent is at least one selected from the group consisting of propylene carbonate, ethylene carbonate, diethyl carbonate, dimethyl carbonate, ethyl methyl carbonate, methyl propyl carbonate, dipropyl carbonate, dimethyl sulfoxide, acetonitrile, dimethoxyethane, diethoxyethane, vinylene carbonate, sulfolane, gamma-butyrolactone, propylene sulfite and tetrahydrofuran.

14. A lithium secondary battery comprising an electrode assembly including a positive electrode, a negative electrode, and a separator disposed between the positive electrode and the negative electrode, and the non-aqueous electrolyte solution for a lithium secondary battery described in claim 10, the electrolyte solution being injected into the electrode structure.
